# FASCICULE DE BREVET EUROPEEN

(11) **EP 0 533 534 B1**
(45) Date de publication et mention de la délivrance du brevet: **18.10.1995**
(21) Numéro de dépôt: 92402468.0
(22) Date de dépôt: 09.09.1992
(51) Int. Cl.: G01B 11/24

(54) **Dispositif d'émission ou d'absorption de lumière pour le contrôle sans contact d'objets**
Gerät zum Absorbieren oder Ausstrahlen von Licht zu berührungslosen Objektvermessung
Device for absorbing or emitting light for contactless testing of objects

(30) Priorité: 11.09.1991 FR 9111227
(43) Date de publication de la demande: 24.03.1993
(73) Titulaire: VERRERIE DU LANGUEDOC ET CIE., F-30310 Vergeze (FR)
(72) Inventeur: Leser, Jacques, F-34400 Lunel (FR)
(74) Mandataire: Casalonga, Axel

(56) Documents cités:
- EP-A- 0 177 004
- EP-A- 0 218 865
- US-A- 4 171 481

## Description

La présente invention concerne un dispositif d'émission ou d'absorption de lumière qui est destiné à être associé à au moins un dispositif optique détecteur comportant une barrette linéaire de photodiodes réceptrices en vue du contrôle sans contact d'objets, en particulier d'objets ou de récipients en verre à haute température.

La production de masse d'objets à grande cadence nécessite l'organisation de procédures de contrôle de qualité efficace et n'entraînant pas des frais excessifs. C'est ainsi en particulier que, pour la fabrication d'objets moulés qui sortent du moule à haute température, on utilise habituellement des dispositifs de contrôle optique.

La demande de brevet français 84 15 117 concerne notamment un procédé et un dispositif permettant un tel contrôle sur des objets en verre fabriqués par injection soufflage. Le contrôle s'effectue en éclairant les récipients et en détectant la lumière traversant le récipient qui se déplace perpendiculairement au dispositif d'éclairage, au moyen d'un barrette linéaire de mesure munie d'une pluralité de photodiodes. Il est prévu dans ce document d'utiliser à titre de source de lumière un tube fluorescent dont l'axe longitudinal est parallèle à celui de la barrette de mesure. Le dispositif comprend un boîtier muni d'une ouverture en forme de fente allongée, encadrée par deux plaques parallèles formant guide de lumière.

De la même manière, le brevet US 4 476 533 prévoit un dispositif de contrôle par transmission de la lumière émise en direction d'une barrette linéaire de photodiodes. Le brevet britannique 2 135 452 prévoit de contrôler des récipients translucides en soumettant ces derniers à une rotation devant un rayon lumineux qui est dirigé par intermittence sur le récipient dans une direction sensiblement parallèle à son axe de rotation. On pourra encore citer le brevet français 2 475 424 et le brevet européen 60 160 qui tous deux utilisent des systèmes optiques que ce soit par l'identification de récipients ou pour le contrôle du verre en feuille.

Dans tous ces documents les sources lumineuses utilisées soit ne sont pas précisées soit sont simplement mentionnées de manière schématique.

Il a été constaté cependant dans la pratique que la structure du dispositif d'éclairage utilisé dans les procédés et dispositifs de contrôle sans contact d'objets, présentait une importance considérable pour la fiabilité du contrôle. Il en est ainsi en particulier dans le cas où le contrôle se fait au moyen d'un dispositif optique de détection muni d'une barrette linéaire de photodiodes réceptrices qui sont disposées selon un alignement orthogonal au déplacement des objets à contrôler.

La présente invention a donc pour objet un dispositif qui puisse être associé à un tel détecteur optique afin d'améliorer la qualité et la fiabilité du contrôle obtenu.

L'invention a également pour objet la réalisation d'un dispositif qui puisse être utilisé dans le cas du contrôle, de l'analyse ou de la reconnaissance de forme d'objets par réflexion ou par transmission de lumière sans aucune modification.

Enfin l'invention a encore pour objet de faciliter le réglage initial de position des différents éléments d'une installation de contrôle d'objets utilisant un détecteur optique muni d'un barrette linéaire de photodiodes réceptrices.

Le dispositif d'émission ou d'absorption de lumière selon l'invention est destiné à être associé à au moins à un dispositif optique détecteur comportant une barrette linéaire de photodiodes réceptrices en vue du contrôle sans contact d'objets, en particulier d'objets en verre à haute température. Selon l'invention, le dispositif comprend un boîtier muni d'une ouverture en forme de fente allongée encadrée par deux plaques parallèles formant guide de lumière. Le boîtier inclut un miroir semi-réfléchissant, un moyen d'éclairage situé derrière le miroir par rapport à la fente et un alignement d'une pluralité de lampes inidividuelles, ledit alignement étant placé de façon que la lumière émise par les sources sensiblement ponctuelles que constituent les lampes individuelles, soit réfléchie par le miroir en direction de la fente allongée.

Il est ainsi possible de procéder à un réglage de position très précis en utilisant les lampes individuelles alignées et en vérifiant leur alignement par rapport à celui de la barrette linéaire de photodiodes réceptrices.

Par ailleurs le dispositif peut être utilisé comme émetteur de lumière en mettant en oeuvre le moyen d'éclairage dont la lumière traverse le miroir semi-réfléchissant et se trouve guidée par les deux plaques parallèles en direction de l'objet à contrôler puis vers le dispositif ou caméra optique de détection muni de la barrette linéaire de photodiodes réceptrices.

On peut également dans un autre mode de réalisation d'une installation de contrôle ou d'analyse de forme, utiliser une autre source de lumière dont la lumière est réfléchie par l'objet à contrôler en direction du dispositif optique de détection. Le dispositif de l'invention joue alors le rôle de dispositif d'absorption de lumière permettant la réalisation d'un champ noir dans l'axe de la barrette linéaire de photodiodes réceptrices derrière l'objet à contrôler et ce grâce au guide de lumière formé par les deux plaques parallèles.

Le moyen d'éclairage comprend avantageusement au moins un tube fluorescent placé parallèlement à la fente allongée dont la longueur est au moins égale à celle de ladite fente. Dans un mode de réalisation préféré le moyen d'éclairage comprend deux tubes fluorescents placés côte à côte de façon à fournir une plage lumineuse sans interruption telle que vue de la fente allongée. Pour obtenir ce résultat il suffit par exemple de faire se chevaucher légèrement les deux tubes fluorescents parallèles.

La largeur de la fente est de préférence choisie la plus faible possible par rapport à l'étendue de la plage lumineuse créée par le moyen d'éclairage. Une telle fente fine combinée à une grande surface d'éclairage à l'intérieur du boîtier du dispositif permet d'obtenir un balayage efficace de l'objet à contrôler devant le dispositif optique de détection.

Pour éviter la pénétration des poussières se trouvant dans l'environnement de l'installation de contrôle, le boîtier est de préférence mis sous pression d'un gaz comprimé par exemple d'air comprimé et comprend à cet effet un orifice d'alimentation en gaz sous pression, ledit gaz s'échappant continuellement par la fente ouverte.

Le miroir semi-réfléchissant est avantageusement de forme allongée et de longueur au moins égale à celle de la fente. Il est placé à 45° par rapport à la fente de façon à permettre à la fois la transmission de la lumière provenant du moyen d'éclairage par transmission et la réflexion de la lumière provenant de l'alignement des lampes individuelles en direction de la fente allongée.

Le dispositif comprend en outre des moyens pour provoquer l'allumage des lampes individuelles indépendamment de celui du moyen d'éclairage.

L'invention sera mieux comprise à l'étude d'un mode de réalisation particulier décrit à titre d'exemple nullement limitatif et illustré par les dessins annexés sur lesquels :
la figure 1 est une vue schématique en coupe vue de dessus d'un dispositif selon l'invention;
la figure 2 est une vue en perspective éclatée des principaux organes du dispositif de l'invention tels qu'ils sont montés à l'intérieur du boîtier, celui-ci n'ayant pas été représenté;
la figure 3 illustre schématiquement l'alimentation électrique du dispositif;
la figure 4 illustre schématiquement un premier mode d'utilisation d'un dispositif selon l'invention; et
la figure 5 illustre schématiquement un autre mode d'utilisation du dispositif de l'invention.

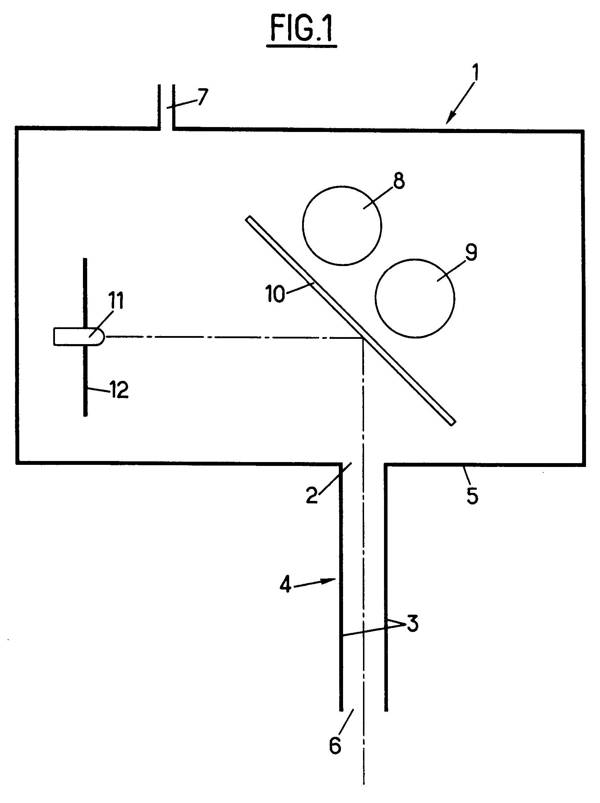

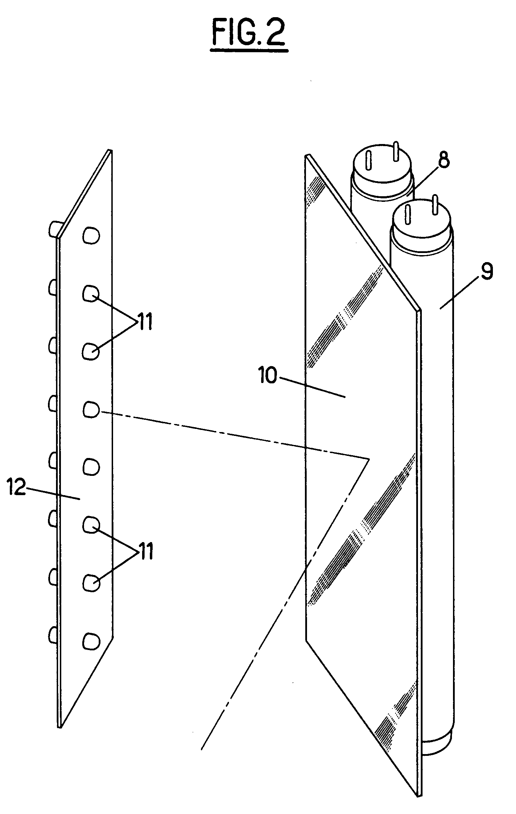

Tel qu'il est illustré sur les figures 1 et 2 le dispositif de l'invention comprend un boîtier 1 muni d'une ouverture en forme de fente allongée 2 qui est encadrée par deux plaques parallèles 3 formant un guide de lumière 4. Les plaques 3 s'étendent perpendiculairement à la face frontale 5 du boîtier 1 et elles définissent au voisinage de leur extrémité libre une nouvelle fente ouverte 6 parallèle à la fente 2. Le boîtier est complètement fermé à part la fente 2 et comporte un orifice 7 permettant l'alimentation en air comprimé sous pression, cet air comprimé s'échappant par la fente 2 puis entre les plaques 3 par la fente 6 et empêchant ainsi toute poussière de pénétrer à l'intérieur du boîtier 1.

A l'intérieur du boîtier 1, se trouvent montés les principaux organes du dispositif. Il s'agit comme on peut voir sur les figures 1 ou 2 d'un moyen d'éclairage constitué par deux tubes fluorescents 8 et 9 montés parallèlement à la fente 2. Les tubes fluorescents 8 et 9 sont de longueur légèrement supérieure à celle de la fente 2 de façon à éclairer complètement celle-ci sur toute sa longueur. Un miroir semi-réfléchissant 10 est monté parallèlement aux tubes 8 et 9 et faisant un angle de 45° avec le plan de la fente 2 c'est-à-dire avec la face frontale 5 du boîtier 1.

Une pluralité de lampes individuelles 11 constituées par des diodes électroluminescentes sont montées en alignement sur une plaque 12 fixée dans le boîtier par des moyens non représentés. La plaque 12 supportant les lampes individuelles 11 est montée dans le boîtier à 45° par rapport au miroir 10. Comme on peut le voir sur les figures 1 et 2, la lumière émise par les sources sensiblement ponctuelles que constituent les lampes individuelles 11 est réfléchie par la face réfléchissante du miroir 10 de façon à pénétrer perpendiculairement dans la fente 2 et le guide de lumière 4. Le trajet lumineux est représenté sur les figures 1 et 2 par un trait mixte.

La lumière émise par les deux tubes fluorescents 8 et 9 traverse quant à elle le miroir 10 et constitue une plage lumineuse large et qui est vue en partie par la fente 2 et depuis l'extérieur par le guide de lumière 4.

Pour que cette plage lumineuse soit sans interruption on dispose les tubes fluorescents 8 et 9 de façon que leur trace telle que vue sur la figure 1 se recouvre légèrement. Une partie du tube lumineux 8 se trouve ainsi derrière le tube fluorescent 9 si l'on regarde l'ensemble depuis la fente 2. Comme on peut le noter sur les figures, les tubes 8 et 9 sont disposés derrière le miroir semi-réfléchissant 10 par rapport à la fente 2 leurs axes définissant un plan parallèle à celui du miroir 10.

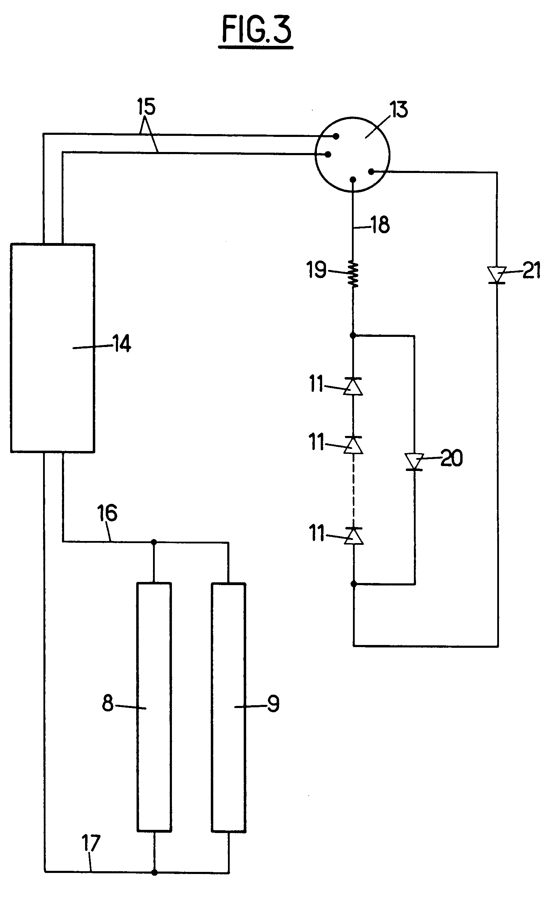

On se reportera maintenant à la figure 3 sur laquelle on a représenté l'alimentation électrique de l'ensemble du dispositif.

Le connecteur 13 situé à l'arrière du boîtier 1 et non représenté sur la figure 1 permet l'alimentation d'un convertisseur tension-fréquence de puissance 14 par l'intermédiaire des connexions 15. Le convertisseur 14 est relié par les connexions 16 et 17 aux deux bornes des tubes fluorescents 8 et 9. Le connecteur 13 est également relié par la connexion 18 à une résistance 19 permettant de limiter le courant électrique, ladite résistance 19 étant reliée aux bornes des différentes diodes électroluminescentes 11 montées en série.

Une diode 20 montée en inverse aux bornes de l'ensemble des diodes électroluminescentes 11 permet d'éviter toute destruction en cas de tension parasite inverse. Une diode supplémentaire 21 est également montée en série entre 12 connecteur 13 et l'ensemble des diodes 11 de façon à éliminer d'éventuelles tensions parasites.

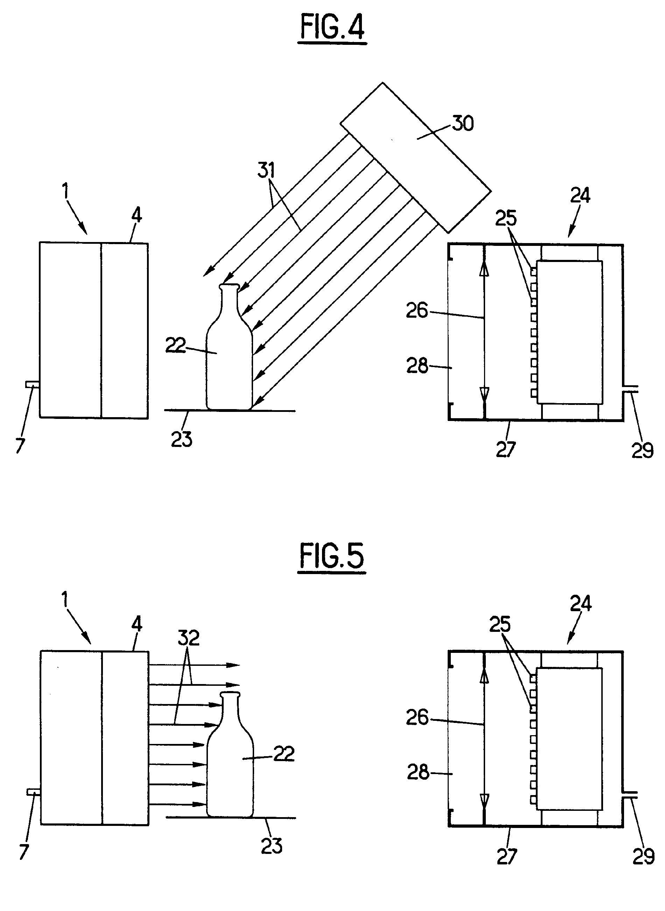

On se référera maintenant aux figures 4 et 5 pour comprendre le mode d'utilisation du dispositif de l'invention.

Sur la figure 4 on utilise le dispositif de l'invention, dont on distingue le boîtier 1 et le guide de lumière 4, comme absorbeur de lumière. L'installation a pour objet le contrôle ou l'analyse et la reconnaissance de forme de récipients en verre 22 qui se déplacent sur un tapis transporteur 23 perpendiculairement à l'installation de contrôle. Celle-ci comprend en plus du dispositif de l'invention, un dispositif optique de détection 24 comportant une barrette linéaire 25 de photodiodes réceptrices placée au foyer d'un dispositif optique 26 de focalisation. L'ensemble est disposé à l'intérieur d'un boîtier 27 muni d'une fente ouverte 28 de forme allongée et disposée parallèlement à la barrette de photodiodes 25 elle-même parallèle à la fente allongée du boîtier 1 du dispositif de l'invention. Le boîtier 27 du dispositif optique de détection est également soumis à la pression d'air comprimé alimenté par l'orifice 29 pour éviter toute pénétration de poussière sur l'optique 26.

Une source d'éclairage supplémentaire 30 est placée au-dessus du dispositif d'optique de détection 24 de façon à émettre un faisceau lumineux 31 en direction du récipient 22 à analyser. D'éventuels défauts ou modifications de l'état de surface du récipient 22 provoquent alors par réflexion un signal lumineux en direction du dispositif optique de détection 24.

Le dispositif de l'invention permet un réglage précis de l'alignement de la caméra du dispositif optique 24 par rapport à l'ensemble de l'installation. Pour opérer cet alignement précis, on effectue tout d'abord un réglage grossier en allumant les tubes fluorescents 8 et 9 situés à l'intérieur du boîtier 1. Puis on éteint les tubes fluorescents et on allume les diodes électroluminescentes 11. On règle alors le dispositif optique de détection 24 de façon à obtenir un alignement précis des lampes électroluminescentes 11 du dispositif de l'invention situées dans le boîtier 1 avec la barrette de photodiodes réceptrices 25. Si cela est nécessaire on provoque une rotation du boîtier 1 pour obtenir l'alignement précis. On éteint ensuite les lampes électroluminescente 11 sans allumer les tubes fluorescents 8 et 9. Le guide de lumière 4 constitué par les plaques parallèles 3 en association avec le boîtier 1 réalise alors un dispositif d'absorption de lumière qui permet de définir un champ noir mince se trouvant exactement aligné avec la barrette de photodiodes 25 et sur lequel on peut observer avec précision les défauts entraînant une déviation de la lumière provenant de la source d'éclairage supplémentaire 30.

Dans le mode de fonctionnement illustré sur la figure 5 où l'on retrouve les mêmes références pour les mêmes organes, on voit que l'installation ne comporte pas de source lumineuse supplémentaire comme c'était le cas dans le mode de réalisation de la figure 4. Le réglage de l'alignement se fait comme précédemment. Après cette opération, on allume les tubes fluorescents 8 et 9 et l'on utilise le dispositif de l'invention 1 comme un dispositif d'éclairage capable de transmettre un faisceau lumineux mince 32 en direction du récipient 22. Les défauts ou l'analyse de forme sont alors détectés par réflexion particulière de la lumière qui atteint les photodiodes réceptrices 25.

Grâce à l'invention on obtient un dispositif extrêmement simple et pratique permettant de faciliter et d'améliorer la fiabilité du système de contrôle ou d'analyse et de reconnaissance de forme d'objets en mouvement et en particulier d'objets en verre à haute température. La contrôle des objets peut s'effectuer par transparence par exemple lorsqu'ils sont en verre ou par réflexion. Dans d'autres applications il est seulement nécessaire d'obtenir le profil de l'objet. Le dispositif de l'invention permet chaque fois de faciliter la détection.

## Revendications

1. Dispositif d'émission ou d'absorption de lumière destiné à être associé à au moins un dispositif optique détecteur (24) comportant une barrette linéaire de photodiodes réceptrices (25) en vue du contrôle sans contact d'objets en particulier d'objets en verre à haute température (22), ledit dispositif d'émission ou d'absorption comprenant un boîtier (1) muni d'une ouverture en forme de fente allongée (2) encadrée par deux plaques parallèles (3) formant guide de lumière (4), caractérisé par le fait que le boîtier (1) inclut un miroir semi-réfléchissant (10), un moyen d'éclairage (8, 9) situé derrière le miroir par rapport à la fente et un alignement d'une pluralité de lampes individuelles (11) placées de façon que la lumière émise par les sources sensiblement ponctuelles que constituent les lampes soit réfléchie par le miroir en direction de la fente.

2. Dispositif selon la revendication 1, caractérisé par le fait que le moyen d'éclairage comprend au moins un tube fluorescent placé parallèlement à la fente et dont la longueur est au moins égale à celle de la fente.

3. Dispositif selon la revendication 2, caractérisé par le fait que le moyen d'éclairage comprend deux tubes fluorescents (8, 9) placés côte à côte de façon à fournir une plage lumineuse sans interruption telle que vue de la fente.

4. Dispositif selon l'une quelconque des revendications précédentes, caractérisé par le fait que la largeur de la fente est faible par rapport à l'étendue de la plage lumineuse créée par le moyen d'éclairage.

5. Dispositif selon l'une quelconque des revendications précédentes, caractérisé par le fait que le boîtier comprend un orifice d'alimentation (7) en gaz sous pression, ledit gaz s'échappant continuellement par la fente pour éviter la pénétration des poussières.

6. Dispositif selon l'une quelconque des revendications précédentes, caractérisé par le fait que le miroir semi-réfléchissant (10) est de forme allongée et de longueur au moins égale à celle de la fente.

7. Dispositif selon l'une quelconque des revendications précédentes, caractérisé par le fait que le miroir semi-réfléchissant est placé à 45° par rapport à la fente.

8. Dispositif selon l'une quelconque des revendications précédentes, caractérisé par le fait qu'il comprend des moyens pour provoquer l'allumage des lampes individuelles indépendamment de celui du moyen d'éclairage.

## Patentansprüche

1. Vorrichtung zum Ausstrahlen bzw. zum Absorbieren von Licht, die dazu bestimmt ist, mindestens einer optischen Erfassungsvorrichtung (24) zugeordnet zu werden, die einen linearen Steg aus Empfangsphotodioden (25) aufweist, bestimmt zur berührungslosen.Vermessung von Objekten, insbesondere von Objekten aus Glas hoher Temperatur (22), wobei diese Vorrichtung zum Ausstrahlen bzw. Absorbieren ein Gehäuse (1) aufweist, das mit einer Öffnung in Form eines länglichen Spalts (2) versehen ist, der von zwei parallelen Platten (3) eingefaßt ist, die eine Lichtführung (4) darstellen, dadurch gekennzeichnet, daß das Gehäuse (1) einen halbreflektierenden Spiegel (10), ein Beleuchtungsmittel (8, 9) hinter dem Spiegel in Bezug auf den Spalt, und eine ausgerichtete Reihe einzelner Lampen (11) aufweist, die so angeordnet sind, daß das von den im wesentlichen punktförmigen Quellen, die diese Lampen darstellen, ausgestrahlte Licht vom Spiegel in Richtung zum Spalt geworfen wird.

2. Vorrichtung gemäß Anspruch 1, dadurch gekennzeichnet, daß das Beleuchtungsmittel wenigstens eine Leuchtstoffröhre umfaßt, die parallel zum Spalt angeordnet ist und deren Länge wenigstens gleich der des Spalts ist.

3. Vorrichtung gemäß Anspruch 2, dadurch gekennzeichnet, daß das Beleuchtungsmittel zwei nebeneinander angeordnete Leuchtstoffröhren (8, 9) umfaßt, so daß sie, vom Spalt aus gesehen, eine nicht unterbrochene Lichtzone darstellen.

4. Vorrichtung gemäß einem beliebigen der vorstehenden Ansprüche, dadurch gekennzeichnet, daß die Breite des Spalts klein ist gegenüber der Ausdehnung der Lichtzone, die durch das Beleuchtungsmittel erzeugt wird.

5. Vorrichtung gemäß einem beliebigen der vorstehenden Ansprüche, dadurch gekennzeichnet, daß das Gehäuse eine Zufuhröffnung (7) für Gas unter Druck aufweist, wobei das Gas kontinuierlich durch den Spalt entweicht, um das Eindringen von Staub zu verhindern.

6. Vorrichtung gemäß einem beliebigen der vorstehenden Ansprüche, dadurch gekennzeichnet, daß der halbreflektierende Spiegel (10) eine längliche Form aufweist und eine Länge hat, die wenigstens gleich der des Spalts ist.

7. Vorrichtung gemäß einem beliebigen der vorstehenden Ansprüche, dadurch gekennzeichnet, daß der halbreflektierende Spiegel im Winkel von 45° zum Spalt angeordnet ist.

8. Vorrichtung gemäß einem beliebigen der vorstehenden Ansprüche, dadurch gekennzeichnet, daß sie Mittel zum Zünden einzelner Lampen unabhängig von dem des Beleuchtungsmittels aufweist.

## Claims

1. Light emission or absorption device intended to be associated with at least one optical detection device (24) including a linear array of reception photodiodes (25) with a view to the contactless inspection of articles , in particular of glass articles at high temperature (22) the said light emission or absorption device comprising a casing (1) equipped with an opening in the form of an elongate slot (2) flanked by two parallel plates (3) forming a light guide (4) characterised in that the casing (1) includes a semi-reflecting mirror (10), an illumination means (8,9) situated behind the mirror with respect to the slot and an alignment of a plurality of individual lamps (11) placed so that the light emitted by the substantially point sources, which the lamps constitute, is reflected by the mirror in the direction of the slot.

2. Device according to Claim 1, characterised in that the illumination means comprises at least one fluorescent tube placed in parallel with the slot and whose length is at least equal to that of the slot .

3. Device according to Claim 2, characterised in that the illumination means comprises two fluorescent tubes (8,9) placed side by side so as to provide a luminous area without interruption as seen from the slot.

4. Device according to anyone of the preceding claims, characterised in that the width of the slot is small with respect to the luminous area created by the illumination means.

5. Device according to any one of the preceding claims, characterised in that the casing comprises an orifice (7) for supply with pressurised gas, the said gas continuously escaping via the slot in order to prevent the penetration of dust.

6. Device according to any one of the preceding claims, characterised in that the semi-reflecting mirror (10) is of elongate shape and of length at least equal to that of the slot.

7. Device according to any one of the preceding claims, characterised in that the semi-reflecting mirror is placed at 45° with respect to the slot.

8. Device according to any one of the preceding claims, characterised in that it comprises means for switching on the individual lamps independently of the switching-on of the illumination means.
